# NEUE EUROPÄISCHE PATENTSCHRIFT

(11) **EP 0 814 098 B2**
(45) Veröffentlichungstag und Bekanntmachung des Hinweises auf die Entscheidung über den Einspruch: **04.09.2013**
(45) Hinweis auf die Patenterteilung: 28.04.2004
(21) Anmeldenummer: 97109943.7
(22) Anmeldetag: 18.06.1997
(51) Int. Cl.: C08F 8/04, C07C 29/20, C08C 19/02

(54) **Verfahren zur Umsetzung einer organischen Verbindung in Gegenwart eines geträgerten Rutheniumkatalysators**
Process for the conversion of an organic compound in the presence of a supported ruthenium-catalyst
Procédé de conversion d'un composé organique en présence d'un catalyseur supporté en ruthénium

(30) Priorität: 19.06.1996 DE 19624485; 21.06.1996 DE 19624835
(43) Veröffentlichungstag der Anmeldung: 29.12.1997
(73) Patentinhaber: BASF SE, 67056 Ludwigshafen (DE)
(72) Erfinder: Rühl, Thomas, Dr., 67227 Frankenthal (DE); Breitscheidel, Boris, Dr., 67117 Limburgerhof (DE); Henkelmann, Jochen, Dr., 68165 Mannheim (DE); Henne, Andreas, Dr., 67433 Neustadt (DE); Lebkücher, Rolf, Dr., 68165 Mannheim (DE); Knoll, Konrad, Dr., 67069 Ludwigshafen (DE); Weiguny, Sabine, Dr., 67251 Freinsheim (DE); Nägele, Paul, 67166 Otterstadt (DE); Gausepohl, Hermann, Dr., 67112 Mutterstadt (DE); Niessner, Norbert, Dr., 67159 Friedelsheim (DE)
(74) Vertreter: Isenbruck, Günter

(56) Entgegenhaltungen:
- EP-A- 0 378 104
- EP-A- 0 653 243
- WO-A-95/25131
- DE-A- 2 845 615
- DE-A- 3 227 650
- DE-A1- 2 823 165
- DE-A1- 2 909 663
- FR-A- 2 344 576

## Beschreibung

Die vorliegende Erfindung betrifft ein Verfahren zur Umsetzung einer organischen Verbindung ausgewählt aus aromatischen Verbindungen in Gegenwart eines Katalysators, der als Aktivmetall/ Ruthenium, aufgebracht auf einem porösen Träger, umfaßt.

In einer Ausführungsform betrifft die vorliegende Erfindung ein Verfahren zur Hydrierung, einer aromatischen Verbindung, in der mindestens eine Hydroxylgruppe an einen aromatischen Kern gebunden ist, wobei vorzugsweise zusätzlich zur mindestes einen Hydroxylgruppe mindestens eine, gegebenenfalls substituierte, C₁₋₁₀-Alkylgruppe und/oder mindestens eine C₁₋₁₀-Alkoxygruppe an einen aromatischen Kern gebunden ist. Des weiteren werden Monoalkyl-substituierte Phenole im erfindungsgemäßen Verfahren bevorzugt eingesetzt.

Die ein- oder mehrkernigen aromatischen Verbindungen werden dabei in Gegenwart des hierin beschriebenen Katalysators vorzugsweise zu den entsprechenden cycloaliphatischen Verbindungen hydriert, wobei die Hydroxylgruppe erhalten bleibt.

Cycloaliphatische Alkohole, insbesondere Alkylcyclohexanole, sind wichtige Zwischenprodukte für die Herstellung verschiedener Duftstoffe, Arzneimittel und anderer organischer Feinchemikalien. Durch katalytische Hydrierung der entsprechenden aromatischen Vorläufer sind die obigen cycloaliphatischen Alkohole bequem zugänglich.

Das Verfahren, Alkylcyclohexanole durch katalytische Hydrierung der entsprechenden Alkylphenole herzustellen, ist bekannt. Die Hydrierung von Alkylphenolen zu den entsprechenden Alkylcyclohexanolen in Gegenwart von Hydrierungskatalysatoren, insbesondere auf Trägern aufgebrachten Katalysatoren, ist vielfach beschrieben.

Als Katalysatoren werden beispielsweise metallisches Rhodium, Rhodium-Platin-, Rhodium-Ruthenium-Legierungen sowie Ruthenium, Palladium oder Nickel auf Katalysatorträgern verwendet. Als Katalysatorträger werden Kohlenstoff, Bariumcarbonat und insbesondere Aluminiumoxid eingesetzt.

In der PL 137 526 ist die Hydrierung von p-tert.-Butylphenol zu p-tert.-Butylcyclohexanol unter Verwendung eines Nickel-Katalysators beschrieben.

In der DE-A-34 01 343 und der EP 0 141 054 ist ein Verfahren zur Herstellung von 2- und 4-tert.-Butylcyclohexanol aus 2- und 4-tert.-Butylphenol durch katalytische Hydrierung beschrieben. Die Hydrierung wird zweistufig ausgeführt, wobei in der ersten Stufe ein Palladium-Katalysator auf einem Al₂O₃-Träger und in der zweiten Stufe ein Ruthenium-Katalysator auf einem Al₂O₃-Träger verwendet wird. Der Metallgehalt auf dem Träger beträgt dabei 0,1 bis 5 Gew.-%. Die Träger sind nicht weiter spezifiziert. Es wird bei einem Druck von 300 bar unter Produktrückführung gearbeitet, und es werden vorzugsweise die cis-tert.-Butylphenole erhalten, wobei 0,1 bis 0,5 % Nebenprodukte anfallen.

Die US 2,927,127 beschreibt ein Verfahren zur Herstellung von p-tert.-Butylcyclohexanol und Estern davon durch katalytische Hydrierung von p-tert.-Butylphenol. Als Katalysatoren werden 5 % Rhodium auf Kohlenstoff, 5 % Palladium auf Bariumcarbonat und 5 % Ruthenium auf Kohlenstoff verwendet. Bei der Verwendung von Ruthenium auf Kohlenstoff wurde mit einem Druck von 70 bis 120 bar und einer Temperatur von 74 bis 93 °C gearbeitet. Als Hydrierungsprodukt wurden 66 % cis-Isomer erhalten.

In der DE-A-29 09 663 ist ein Verfahren zur Herstellung von cis-Alkylcyclohexanolen durch katalytische Hydrierung der entsprechenden Alkylphenole beschrieben. Als Katalysator wurde Ruthenium auf einem Al₂O₃-Träger verwendet. Es wurde bei Drücken von 40, 60 oder 80 bar gearbeitet. Als Produkt wurden überwiegend cis-Alkylcyclohexanole erhalten, wobei als Nebenprodukt 0,1 bis 1 % Alkylbenzole anfielen.

In einer weiteren Ausführungsform betrifft die vorliegende Erfindung ein Verfahren zur Hydrierung, einer aromatischen Verbindung, in der mindestens eine Aminogruppe an einen aromatischen Kern gebunden ist, wobei vorzugsweise zusätzlich zur mindestens einen Aminogruppe mindestens eine, ggf. substituierte, C₁₋₁₀-Alkylgruppe und/oder mindestens eine C₁₋₁₀-Alkoxygruppe an einen aromatischen Kern gebunden ist. Insbesondere bevorzugt werden Monoallyl-substituierte Amine eingesetzt.

Die ein- oder mehrkernigen aromatischen Verbindungen werden dabei in Gegenwart des hierin beschriebenen Katalysators vorzugsweise zu den entsprechenden cycloaliphatischen Verbindungen hydriert, wobei die Aminogruppe erhalten bleibt.

Cycloaliphatische Amine, insbesondere gegebenenfalls substituierte Cyclohexylamine und Dicyclohexylamine, finden Verwendung zur Herstellung von

Alterungsschutzmitteln für Kautschuke und Kunststoffe, als Korrosionsschutzmittel sowie als Vorprodukte für Pflanzenschutzmittel und Textilhilfsmittel. Cycloaliphatische Diamine werden zudem bei der Herstellung von Polyamid- und Polyurethanharzen eingesetzt und finden weiterhin Verwendung als Härter für Epoxidharze.

Es ist bekannt, cycloaliphatische Amine durch katalytische Hydrierung der entsprechenden ein- oder mehrkernigen aromatischen Amine herzustellen. Die Hydrierung von aromatischen Aminen zu den entsprechenden cycloaliphatischen Aminen in Gegenwart von Hydrierungskatalysatoren, insbesondere auf Trägern aufgebrachten Katalysatoren, ist vielfach beschrieben.

Als Katalysatoren werden beispielsweise Raney-Kobalt mit basischen Zusätzen (JP 43/3180), Nickel-Katalysatoren (US 4,914,239, DE 80 55 18), RhodiumKatalysatoren (BE 73 93 76, JP 70 19 901, JP 72 35 424), sowie Palladiumkatalysatoren (US 3,520,928, EP 501 265, EP 53 818, JP 59/196 843) verwendet. In der Mehrzahl werden jedoch rutheniumhaltige Katalysatoren eingesetzt.

Aus der DE 21 32 547 ist ein Verfahren zur Hydrierung ein- oder mehrkerniger aromatischer Diamine zu den entsprechenden cycloaliphatischen Aminen bekannt, das in Gegenwart eines suspendierten Rutheniumkatalysators ausgeführt wird.

In der EP 67 058 ist ein Verfahren zur Herstellung von Cyclohexylamin durch katalytische Hydrierung des entsprechenden aromatischen Amins beschrieben. Als Katalysator wird Rutheniummetall in einer fein verteilten Form auf aktivierten Aluminiumpellets verwendet. Nach vier Rückführungen begann der Katalysator seine Wirksamkeit zu verlieren.

Die EP 324 984 betrifft ein Verfahren zur Herstellung eines Gemisches aus gegebenenfalls substituiertem Cyclohexylamin und gegebenenfalls substituiertem Dicyclohexylamin durch Hydrierung von gegebenenfalls substituiertem Anilin unter Verwendung eines Ruthenium und Palladium auf einem Träger enthaltenden Katalysators, der darüber hinaus eine alkalisch reagierende Alkalimetallverbindung als Modifiziermittel enthält. Ein prinzipiell ähnliches Verfahren ist in der EP 501 265 beschrieben, wobei dort der Katalysator als Modifiziermittel Niobsäure, Tantalsäure oder ein Gemisch beider enthält.

In der US 2,606,925 wird ein Verfahren zur Herstellung einer Aminocyclohexyl-Verbindung durch Hydrieren einer entsprechenden aromatischen Verbindung beschrieben, wobei ein Rutheniumkatalysator, dessen aktive katalytische Komponente ausgewählt wird unter elementarem Ruthenium, Rutheniumoxiden, Rutheniumsalzen, in denen das Ruthenium im Anion oder im Kation vorhanden ist. Wie die Beispiele dieses Verfahrens zeigen, wird auch dort der Katalysator in einer getrennten Stufe hergestellt und getrocknet und nach längerer Trocknungszeit in das Reaktionsgefäß eingebracht.

Ein weiteres Verfahren zur Herstellung von Cyclohexylamin wird in der US 2,822,392 beschrieben, wobei das Hauptaugenmerk dieser Patentschrift auf die Verwendung eines speziellen Reaktors, in dem das Anilin und der Wasserstoff als Ausgangsprodukte im Gegenstrom miteinander zur Umsetzung gebracht werden, gerichtet ist.

Die US 3,636,108 und US 3,697,449 betreffen die katalytische Hydrierung aromatischer, Stickstoff enthaltender Verbindung unter Verwendung eines Rutheniumkatalysators, der zusätzlich eine Alkalimetallverbindung als Modifiziermittel umfaßt.

Allen oben beschriebenen Verfahren gemeinsam ist die Verwendung von mesoporösen Trägern mit BET-Oberflächen, die typischerweise zwischen 50 und über 1000 m²/g liegen, um eine hohe Aktivität des Katalysators zu erzielen.

Ferner hat es sich, insbesondere bei der Hydrierung mit einem rhodiumhaltigen Katalysator, neben dem hohen Preis für den Katalysator, als nachteilig erwiesen, daß bei diesen Umsetzungen nicht selten größere Mengen an Alkylbenzolen sowie weiteren, nicht identifizierbaren Verbindungen, die bei der Hydrierung als Zersetzungs- bzw. Nebenprodukte gebildet werden, auftraten. Diese Nebenprodukte erschweren die Aufarbeitung und Reinigung des Reaktionsproduktes insbesondere dann, wenn z. B. Alkylcyclohexanole als Duftstoffe bzw. zur Herstellung von Duftstoffen verwendet werden sollen. Ferner nimmt die Aktivität vieler der in den oben beschriebenen Verfahren verwendete Katalysatoren rasch ab, und zwar insbesondere dann, wenn die Hydrierung zur Beschleunigung der Reaktionsgeschwindigkeit bei höheren Reaktionstemperaturen durchgeführt wird.

Verfahren zur Hydrierung von Polymeren, die mindestens eine hydrierbare Einheit aufweisen, sind an sich bekannt.

Ein Katalysator, bestehend aus einem makroporösen Trägermaterial, auf das ein Metall der VIII. Nebengruppe des Periodensystems aufgebracht ist, der zur Hydrierung von Kohlenstoff-Kohlenstoff-Doppelbindungen verwendet werden kann, ist in der US 5,110,779 beschrieben. Neunzig Prozent der im Trägermaterial vorhandenen Poren des dort beschriebenen Katalysators besitzen einen Durchmesser von mehr als 100 nm. Das Verhältnis der Oberflächen von Metall zu Träger beträgt dabei 0,07 bis 0,75:1. Dabei wird im Rahmen dieser Patentschrift insbesondere auf die große Oberfläche des Metalls im Verhältnis zum Träger abgehoben, und als überraschend angegeben, da ein derartiger Katalysator noch über eine hohe Aktivität verfügt.

EP-A 0 653 243 betrifft Trägerkatalysatoren, die eine aktive Komponente aufweisen, die sich zum überwiegenden Teil in Meso- und Makroporen befindet. Bei der katalytisch aktiven Komponente handelt es sich im Allgemeinen um Pd-, Pt-, Ag-, Cu-, Zn-, Fe-, Cr-, Ni-, Mn-, Co-, Rh-, Ru-, Re- und Os-Verbindungen. Diese Trägerkatalysatoren werden zur Hydrierung ungesättigter organischer Verbindungen verwendet. Gemäß der Beschreibung wird die Verwendung der Trägerkatalysatoren unter anderem zur selektiven Hydrierung erwähnt.

Eine Aufgabe der vorliegenden Erfindung lag demgemäß in der Bereitstellung eines Verfahrens zur Hydrierung, einer organischen Verbindung, wie eingangs definiert, wobei sehr hohe Ausbeuten beziehungsweise nahezu vollständiger Umsatz erreicht werden.

Eine weitere Aufgabe der Erfindung liegt in der Bereitstellung eines derartigen Verfahrens, wobei lediglich ein minimaler Anteil von Nebenprodukten beziehungsweise Zersetzungsprodukten während der Hydrierung anfällt.

Ferner sollte es möglich sein, das Verfahren unter hohen Katalysatorbelastungen und langen Standzeiten mit einer extrem hohen Turn-Over-Zahl durchzuführen, wobei die entsprechenden Hydrierungsprodukte in hoher Ausbeute und hoher Reinheit erhalten werden.

Gelöst werden eine oder mehrere der vorstehenden Aufgaben durch ein Verfahren mit den Merkmalen des Anspruchs 1.

Die vorstehenden Aufgaben sowie gegebenenfalls weitere Aufgaben werden durch Verfahren zur Hydrierung, wie sie in den Unteransprüchen beschreiben sind, gelöst. Ein besonderer Vorteil der erfindungsgemäßen Verfahren liegt darin, daß sehr gute Ergebnisse bei der Verwendung von lediglich geringen Metallgehalten im Katalysator erzielt werden.

Ferner weisen die erfindungsgemäßen Verfahren hohe Turn-Over-Zahlen bei hoher Katalysatorbelastung und über lange Katalysatorstandzeiten hinweg auf. Die Katalysatorbelastung ist dabei die Raum/Zeit-Ausbeute des Verfahrens, d. h. die Menge des umgesetzten Edukts pro Zeiteinheit und pro Menge an vorliegendem Katalysator. Standzeit bedeutet die Zeit bzw. die Menge an umgesetztem Edukt, die ein Katalysator verkraftet, ohne seine Eigenschaften einzubüßen und ohne daß sich die Produkteigenschaften signifikant verändern.

### VERBINDUNGEN

Besonders geeignet ist das erfindungsgemäße Verfahren zur Hydrierung einer organischen Verbindung, die ausgewählt wird aus der Gruppe bestehend aus einer aromatischen Verbindung, in der mindestens eine Hydroxylgruppe an einen aromatischen Kern gebunden ist, einer aromatischen Verbindung, in der mindestens eine Aminogruppe an einen aromatischen Kern gebunden ist, und einem Gemisch aus zwei oder mehr davon.

Im Rahmen des erfindungsgemäßen Verfahrens können auch organische Verbindungen umgesetzt werden, die Einheiten verschiedener Strukturen

C=C (I)

C≡C (II)

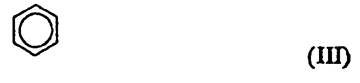

C=N (IV)

C≡N (V)

C=O (VI)

C=S (VII)

―NO₂ (VIII)

enthalten, z. B. organische Verbindungen, die sowohl C-C-Mehrfachbindungen als auch Carbonylgruppen aufweisen, da die im Rahmen des erfindungsgemäßen Verfahrens verwendeten Katalysatoren in der Lage sind, zunächst eine der beiden Gruppen selektiv zu hydrieren, d. h. eine Hydrierung dieser Gruppen von ungefähr 90 bis 100 % zu erreichen, während zunächst gleichzeitig die anderen Gruppen zu weniger als 25 % und im allgemeinen in einem Bereich von 0 bis ungefähr 7 % hydriert werden. Dabei werden im allgemeinen zunächst die C-C-Mehrfachbindungen und anschließend die C=O-Gruppen hydriert.

Der Begriff "aromatische Verbindung, in der mindestens eine Hydroxylgruppe an einem aromatischen Kern gebunden ist" bzw. "aromatische Verbindung, in der mindestens eine Aminogruppe an einen aromatischen Kern gebunden ist" steht für alle Verbindungen, die eine Einheit der folgenden Struktur (I) aufweisen: 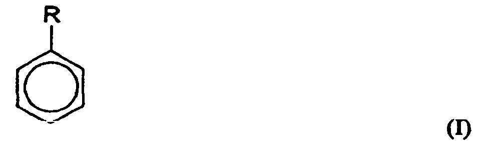 wobei R eine Hydroxyl- oder eine Aminogruppe darstellt.

Sofern im Rahmen des erfindungsgemäßen Verfahrens aromatische Verbindungen eingesetzt werden, in denen mindestens eine Hydroxylgruppe und ferner mindestens ein gegebenenfalls substituierter C₁₋₁₀-Alkylrest und/oder -Alkoxyrest an einen aromatischen Kern gebunden ist, kann je nach Reaktionsbedingungen (Temperatur, Lösungsmittel) das erhaltene Isomerenverhältnis von cis- zu trans-konfigurierten Produkten in einem weiten Bereich variiert werden. Ferner können die erhaltenen Verbindungen ohne weitere Reinigungsschritte weiterverarbeitet werden. Dabei wird die Bildung von Alkylbenzolen praktisch vollständig vermieden.

Wie auch die oben beschriebenen Verbindungen, in denen mindestens eine Hydroxylgruppe an einen aromatischen Kern gebunden ist, können im Rahmen des erfindungsgemäßen Verfahrens auch aromatische Verbindungen, in denen mindestens eine Aminogruppe an einen aromatischen Kern gebunden ist, mit hoher Selektivität zu den entsprechenden cycloaliphatischen Verbindungen hydriert werden, wobei für die zusätzlich mit einem C₁₋₁₀-Alkylrest und/oder -Alkoxyrest substituierten Amine bezüglich des Verhältnisses der cis- und trans-Isomeren das oben Gesagte gilt.

Insbesondere wird im Rahmen dieser Ausführungsform die Bildung von Desaminierungsprodukten, wie beispielsweise Cyclohexanen oder teilhydrierten Dimerisierungsprodukten wie Phenylcyclohexylaminen, praktisch vollständig vermieden.

Im einzelnen können im Rahmen des erfindungsgemäßen Verfahrens folgende Verbindungen umgesetzt werden:

### Aromatische Verbindungen, in denen mindestens eine Hydroxylgruppe an einen aromatischen Kern gebunden ist

Mit dem erfindungsgemäßen Verfahren können aromatische Verbindungen, in denen mindestens eine Hydroxylgruppe und vorzugsweise ferner mindestens ein gegebenenfalls substituierter C₁₋₁₀-Alkylrest und/oder Alkoxyrest an einen aromatischen Kern gebunden ist, umgesetzt, vorzugsweise zu den entsprechenden cycloaliphatischen Verbindungen hydriert werden, wobei auch Gemische zweier oder mehr dieser Verbindungen eingesetzt werden können. Dabei können die aromatischen Verbindungen einkernige oder mehrkernige aromatische Verbindungen sein. Die aromatischen Verbindungen enthalten mindestens eine Hydroxylgruppe, die an einen aromatischen Kern gebunden ist, die einfachste Verbindung dieser Gruppe ist Phenol. Vorzugsweise weisen die aromatischen Verbindungen eine Hydroxylgruppe pro aromatischem Kern auf. Die aromatischen Verbindungen können an dem aromatischen Kern oder den aromatischen Kernen substituiert sein durch einen oder mehrere Alkyl- und/oder Alkoxyreste, vorzugsweise C₁₋₁₀-Alkyl- und/oder Alkoxyreste, besonders bevorzugt C₁₋₁₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, wie z.B. Methoxy-, Ethoxy-, Propoxy-, Isopropoxy-, Butoxy-, Isobutoxy-, tert.-Butoxy-Reste, bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Vorzugsweise weisen die erfindungsgemäß umsetzbaren, vorzugsweise hydrierbaren Verbindungen mindestens einen, vorzugsweise einen bis vier, insbesondere einen C₁₋₁₀-Alkylrest auf, der sich vorzugsweise am gleichen aromatischen Kern befindet wie die mindestens eine Hydroxylgruppe. Bevorzugte Verbindungen sind (Mono)alkylphenole, wobei der Alkylrest in o-, m- oder p-Position zur Hydroxylgruppe stehen kann. Insbesondere bevorzugt sind trans-Alkylphenole, auch als 4-Alkylphenole bezeichnet, wobei der Alkylrest vorzugsweise 1 bis 10 Kohlenstoffatome aufweist und insbesondere ein tert.-Butylrest ist. Bevorzugt ist 4-tert.-Butylphenol. Erfindungsgemäß verwendbare mehrkernige aromatische Verbindungen sind beispielsweise β-Naphthol und α-Naphthol.

Die aromatischen Verbindungen, in denen mindestens eine Hydroxylgruppe und vorzugsweise ferner mindestens ein gegebenenfalls substituierter C₁₋₁₀-Alkylrest und/oder Alkoxyrest an einen aromatischen Kern gebunden ist, können auch mehrere aromatische Kerne aufweisen, die über einen Alkylenrest, vorzugsweise eine Methylengruppe verknüpft sind. Die verknüpfende Alkylengruppe, vorzugsweise Methylengruppe, kann einen oder mehrere Alkylsubstituenten aufweisen, die C₁₋₂₀-Alkylreste sein können und vorzugsweise C₁₋₁₀-Alkylreste, besonders bevorzugt sind Methyl-, Ethyl-, Propyl-, Isopropyl-, Butyl- oder tert.-Butylreste, sind.

Dabei kann jeder der aromatischen Kerne mindestens eine Hydroxylgruppe gebunden enthalten. Beispiele solcher Verbindungen sind Bisphenole, die in 4-Position über einen Alkylenrest, vorzugsweise einen Methylenrest, verknüpft sind.

Insbesondere bevorzugt umgesetzt wird im Rahmen des erfindungsgemäßen Verfahrens ein mit einem C₁₋₁₀-Alkylrest, vorzugsweise C₁₋₆-Alkylrest, substituiertes Phenol, wobei der Alkylrest gegebenenfalls mit einem aromatischen Rest substituiert ist, oder Gemische zweier oder mehrerer dieser Verbindungen.

In einer weiteren bevorzugten Ausführungsform dieses Verfahrens wird p-tert.-Butylphenol, Bis(p-hydroxyphenyl)dimethylmethan oder ein Gemisch davon umgesetzt.

### Aromatische Verbindungen, in denen mindestens eine Aminogruppe an einen aromatischen Kern gebunden ist

Mit dem erfindungsgemäßen Verfahren können ferner aromatische Verbindungen, in denen mindestens eine Aminogruppe an einen aromatischen Kern gebunden ist, umgesetzt, vorzugsweise zu den entsprechenden cycloaliphatischen Verbindungen hydriert werden, wobei auch Gemische zweier oder mehr dieser Verbindungen eingesetzt werden können. Dabei können die aromatischen Verbindungen einkernige oder mehrkernige aromatische Verbindungen sein. Die aromatischen Verbindungen enthalten mindestens eine Aminogruppe, die an einen aromatischen Kern gebunden ist. Vorzugsweise sind die aromatischen Verbindungen aromatische Amine oder Diamine. Die aromatischen Verbindungen können an dem aromatischen Kern oder den aromatischen Kernen oder an der Aminogruppe substituiert sein durch einen oder mehrere Alkyl- und/oder Alkoxyreste, vorzugsweise C₁₋₂₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, wie z.B. Methoxy-, Ethoxy-, Propoxy-, Isopropoxy-, Butoxy-, Isobutoxy-, tert.-Butoxy-Reste, bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Die aromatische Verbindung, in der mindestens eine Aminogruppe an einen aromatischen Kern gebunden ist, kann auch mehrere aromatische Kerne aufweisen, die über eine Alkylengruppe, vorzugsweise eine Methylengruppe, verknüpft sind. Die verknüpfende Alkylengruppe, vorzugsweise Methylengruppe, kann einen oder mehrere Alkylsubstituenten aufweisen, die C₁₋₂₀-Alkylreste sein können und vorzugsweise C₁₋₁₀-Alkylreste, besonders bevorzugt Methyl-, Ethyl-, Propyl-, Isopropyl-, Butyl-, sec.-Butyl- oder tert.-Butylreste, sind.

Die an den aromatischen Kern gebundene Aminogruppe kann ebenfalls durch einen oder zwei der vorstehend beschriebenen Alkylreste substituiert sein.

Besonders bevorzugte Verbindungen sind Anilin, Naphthylamin, Diaminobenzole, Diaminotoluole und Bis-p-aminophenylmethan oder Gemische davon.

Aromatische Monomere schließen mono- und polyvinylyubstituierte aromatische Verbindungen ein, wobei Styrol, α-Methylstyrol, Acrylnitril, Methacrylnitril und Divinylbenzol vorzugsweise eingesetzt werden. Darüber hinaus können auch Mischungen von vinylaromatischen und Diolefin-Monomeren, gegebenenfalls zusammen mit herkömmlichen olefinischen Monomeren.

### Katalysatoren

Die erfindungsgemäß verwendeten Katalysatoren können technisch hergestellt werden durch Auftragen von Ruthenium auf einem geeigneten Träger. Die Auftragung kann durch Tränken des Trägers in wäßrigen Metallsalzlösungen, wie Ruthenium salzlösungen, durch Aufsprühen entsprechender Metallsalzlösungen auf den Träger oder durch andere geeignete Verfahren erreicht werden. Als Ruthenium salze zur Herstellung der Ruthenium salzlösungen eignen sich die Nitrate, Nitrosylnitrate, Halogenide, Carbonate, Carboxylate, Acetylacetonate, Chlorkomplexe, Nitritokomplexe oder Aminkomplexe der entsprechenden Metalle, wobei die Nitrate und Nitrosylnitrate bevorzugt sind.

Die mit der Rutheniumsalz lösung beschichteten bzw. getränkten Träger werden anschließend getrocknet, wobei Temperaturen zwischen 100°C und 150°C bevorzugt sind. Wahlweise können diese Träger bei Temperaturen zwischen 200°C und 600°C, vorzugsweise 350°C bis 450°C calciniert werden. Anschließend werden die beschichteten Träger durch Behandlung in einem Gasstrom, der freien Wasserstoff enthält, bei Temperaturen zwischen 30°C und 600°C, vorzugsweise zwischen 150°C und 450°C aktiviert. Der Gasstrom besteht vorzugsweise aus 50 bis 100 Vol.-% H₂ und 0 bis 50 Vol.-% N₂.

Die Ruthenium salzlösung wird in einer solchen Menge auf den/die Träger aufgebracht, daß der Gehalt an Aktivmetall 0,01 bis 30 Gew.-%, vorzugsweise 0,01 bis 10 Gew.-% und insbesondere 0,01 bis 5 Gew.-%, bezogen auf das Gesamtgewicht des Katalysators, an Ruthenium auf den Träger aufgebracht, vorliegen.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J. LeMaitre et al., "Characterization of Heterologous Catalysts ", Hrsg. Francis Delanney, Marcel Dekker, New York (1984), S. 310 - 324, beschrieben ist.

Im erfindungsgemäß verwendeten Katalysator beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

### TRÄGER

Die zur Herstellung der erfindungsgemäß verwendeten Katalysatoren verwendbaren Trägermaterialien besitzen Makroporen und Mesoporen. Dabei bezeichnet der Begriff *"Makroporen"* Poren, deren Durchmesser 50 nm übersteigt, und der Begriff *"Mesoporen"* bezeichnet Poren mit einem mittleren Durchmesser zwischen ungefähr 2,0 nm und ungefähr 50 nm, wie sich aus der Definition in Pure Applied Chem. 45, S. 71ff, insbesondere S. 79 (1976) ergibt.

Dabei weisen die erfindungsgemäß verwendbaren Träger eine Porenverteilung auf, der gemäß ungefähr 10 bis ungefähr 50%, vorzugsweise ungefähr 15 bis ungefähr 50%, weiter bevorzugt 15 bis 45% und insbesondere 30 bis 40% des Porenvolumens von Makroporen mit Porendurchmesser im Bereich von ungefähr 50 nm bis ungefähr 10.000 nm und ungefähr 50 bis ungefähr 90%, vorzugsweise ungefähr 50 bis ungefähr 85%, weiter bevorzugt ungefähr 55 bis ungefähr 85% und insbesondere ungefähr 60 bis ungefähr 70% des Porenvolumens von Mesoporen mit einem Porendurchmesser von ungefähr 2 bis ungefähr 50 nm gebildet werden, wobei sich jeweils die Summe der Porenvolumina zu 100% addiert.

Vorzugsweise beträgt die Oberfläche des Trägers ungefähr 50 bis ungefähr 500 m²/g, weiter bevorzugt ungefähr 200 bis ungefähr 350 m²/g und insbesondere ungefähr 200 bis ungefähr 250 m²/g des Trägers.

Die Oberfläche des Trägers wird nach dem BET-Verfahren durch N₂-Adsorption, insbesondere nach DIN 66131, bestimmt. Die Bestimmung des mittleren Porendurchmesser und der Größenverteilung erfolgt durch Hg-Porosymmetrie, insbesondere nach DIN 66133.

Die erfindungsgemäß verwendeten Katalysatoren zeigen eine hohe Reaktivität (hohe Turn-Over-Zahl), Selektivität und Standzeit. Unter Einsatz der erfindungsgemäß verwendeten Katalysatoren werden in der Hydrierung die Hydrierungsprodukte in hoher Ausbeute und Reinheit gewonnen, wobei eine nachfolgende Reinigung überflüssig ist. Der Umsatz ist praktisch quantitativ. Das erhaltene Hydrierungsprodukt kann in einer bevorzugten Ausführungsform der vorliegenden Erfindung somit direkt einer Weiterverarbeitung zugeführt werden, ohne gereinigt werden zu müssen.

Bei der Hydrierung einer aromatischen Verbindung, in der mindestens eine Hydroxylgruppe an einen aromatischen Kern gebunden ist, werden insbesondere bei der Hydrierung von 4-Alkyl- oder 4-Alkoxy-substituierten Phenolen, wie sie vorstehend beschrieben sind, überwiegend trans-cycloaliphatische Verbindungen erhalten. Der Anteil an trans-cycloaliphatischen Verbindungen Beträgt dabei gemäß einer Ausführungsform der Erfindung mindestens 60 %, vorzugsweise mindestens 65 %. Der Umsatz ist praktisch quantitativ, der Restaromatenteil liegt vorzugsweise unter 0,01 Gew.-%, bezogen auf die gesamte Produktmenge. Das erhaltene Hydrierungsprodukt kann in einer bevorzugten Ausführungsform der vorliegenden Erfindung somit direkt einer Weiterverarbeitung zugeführt werden, ohne gereinigt werden zu müssen.

### Lösungs- oder Verdünnungsmittel

Beim erfindungsgemäßen Verfahren kann die Hydrierung, unter Abwesenheit eines Lösungs- oder Verdünnungsmittels durchgeführt werden, d. h. es ist nicht erforderlich, die Umsetzung in Lösung durchzuführen.

Es kann auch die Schmelze eines Polymers direkt umgesetzt werden.

Vorzugsweise wird jedoch ein Lösungs- oder Verdünnungsmittel eingesetzt. Als Lösungs- oder Verdünnungsmittel kann jedes geeignete Lösungs- oder Verdünnungsmittel eingesetzt werden. Die Auswahl ist dabei nicht kritisch. Beispielsweise können die Lösungs- oder Verdünnungsmittel auch geringe Mengen an Wasser enthalten.

Bei der Hydrierung, einer aromatischen Verbindung, in der mindestens eine Hydroxylgruppe an einen aromatischen Kern gebunden ist, schließen Beispiele geeigneter Lösungs- oder Verdünnungsmittel die folgenden ein:

Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome, aufweist.

Beispiele bevorzugt verwendbarer Alkohole sind i-Propanol, n-Butanol, i-Butanol und n-Hexanol.

Gemische dieser oder anderer Lösungs- oder Verdünnungsmittel können ebenfalls verwendet werden.

Bei der Hydrierung, einer aromatischen Verbindung, in der mindestens eine Aminogruppe an einen aromatischen Kern gebunden ist, schließen Beispiele geeigneter Lösungs- oder Verdünnungsmittel die folgenden ein:

Geradkettige oder cyclische Ether, wie beispielsweise Tetrahydrofuran oder Dioxan, sowie Ammoniak und Mono- oder Dialkylamine, in denen der Alkylrest vorzugsweise 1 bis 3 Kohlenstoffatome aufweist, wie z. B. Methyl-, Ethyl-, Propylamin oder die entsprechenden Dialkylamine.

Gemische dieser oder anderer Lösungs- oder Verdünnungsmittel können ebenfalls verwendet werden.

In beiden obigen Ausführungsformen ist die Menge des eingesetzten Lösungs- oder Verdünnungsmittels nicht in besonderer Weise beschränkt und kann je nach Bedarf frei gewählt werden, wobei jedoch solche Mengen bevorzugt sind, die zu einer 10 bis 70 Gew.-%igen Lösung der zur Hydrierung vorgesehenen Verbindung führen.

Besonders bevorzugt wird im Rahmen des erfindungsgemäßen Verfahrens das bei der Hydrierung gebildete Produkt als Lösungsmittel eingesetzt, gegebenenfalls neben anderen Lösungs- oder Verdünnungsmitteln. In diesem Fall kann ein Teil des im Verfahren gebildeten Produktes den zu hydrierenden Verbindungen beigemischt werden. Bezogen auf das Gewicht der zur Umsetzung, vorzugsweise Hydrierung, vorgesehenen aromatischen Verbindungen wird vorzugsweise die 1 bis 30-fache, besonders bevorzugt die 5 bis 20-fache, insbesondere die 5 bis 10-fache Menge des Umsetzungsproduktes als Lösungs- oder Verdünnungsmittel zugemischt.

Auch für die anderen erfindungsgemäß umsetzbaren Verbindungen gilt das oben Gesagte, wobei auch dort keine Beschränkungen bezüglich der Lösungs- und Verdünnungsmittel bestehen.

Bei der Hydrierung von Polymeren schließen Beispiele geeigneter Lösungs- oder Verdünnungsmittel die folgenden ein:

Kohlenwasserstoffe, wie z. B. Hexan, Cyclohexan, Methylcyclohexan, Heptan, Octan, Toluol, Xylol usw., und geradkettige oder cyclische Ether, wie z. B. Tetrahydrofuran, Dioxan, Dibutylether, Methyl-tert.-Butylether usw., Ketone, wie z. B. Methylethylketon und Aceton, Ester, wie z. B. Ethylacetat, oder Amide, wie z. B. DMF und N-Methylpyrrolidon.

Vorzugsweise werden Cyclohexan, Toluol oder THF eingesetzt. Gemische dieser und anderer Lösungs- und Verdünnungsmittel können ebenfalls verwendet werden.

Sofern das Polymer durch Lösungspolymerisation erhalten wurde, kann die das Polymer enthaltende resultierende Lösung direkt zur Umsetzung im erfindungsgemäßen Verfahren eingesetzt werden.

Die Menge des eingesetzten Lösungs- oder Verdünnungsmittels ist im Rahmen des erfindungsgemäßen Verfahrens nicht in besonderer Weise beschränkt und kann je nah Bedarf frei gewählt werden, wobei jedoch solche Mengen bevorzugt sind, die zu einer 1 bis 70-%igen,vorzugsweise 1 bis 40 Gew.-%igen Lösung, des zur Umsetzung vorgesehenen Polymers führen.

### Umsetzung

Die Hydrierung wird bei geeigneten Drücken und Temperaturen durchgeführt. Bevorzugt sind Drücke oberhalb von ungefähr 2 x 10⁶ Pa, vorzugsweise oberhalb von ungefähr 5 x 10⁶ Pa, insbesondere von ungefähr 1 x 10⁷ bis ungefähr 3 x 10⁷ Pa. Bevorzugte Temperaturen liegen in einem Bereich von ungefähr 30 bis ungefähr 250 °C, vorzugsweise von ungefähr 100 bis ungefähr 220 °C und insbesondere bei ungefähr 150 °C bis ungefähr 200 °C.

Das Hydrierungsverfahren kann kontinuierlich oder in Art eines Batch-Verfahrens durchgeführt werden. Beim kontinuierlichen Verfahren kann dabei ein Teil des den Reaktor verlassenden Hydrierungsproduktes dem Reaktorzulauf vor dem Reaktor zugeführt werden. Dabei wird eine solche Menge des den Reaktor verlassenden Hydrierungsprodukts als Lösungsmittel rückgeführt, daß die im Unterabschnitt "Lösungs- und Verdünnungsmittel" genannten Mengenverhältnisse erreicht werden. Die verbleibende Menge an Hydrierungsprodukt wird abgezogen.

Bei kontinuierlicher Prozeßführung beträgt die Menge der zur Hydrierung vorgesehenen Verbindung bzw. Verbindungen vorzugsweise ungefähr 0,05 bis ungefähr 3 kg pro Liter Katalysator pro Stunde, weiter bevorzugt ungefähr 0,1 bis ungefähr 1 kg pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Je nach Reaktionsbedingungen kann bei den zusätzlich durch mindestens einen ggf. substituierten C₁₋₁₀- und/oder -Alkoxyrest substituierten Phenolen und Aminen je nach Reaktionsbedingungen (Temperatur, Lösungsmittel) das erhaltene Isomerenverhältnis von cis- zu trans-konfigurierten Produkten in einem weiten Bereich variiert werden.

Sofern eine aromatische Verbindung, in der mindestens eine Aminogruppe an einen aromatischen Kern gebunden ist, mittels des erfindungsgemäßen Katalysators hydriert werden soll, kann die Hydrierung auch in Gegenwart von Ammoniak oder Dialkylaminen, beispielsweise Methylamin, Ethylamin, Propylamin oder Dimethylamin, Diethylamin oder Dipropylamin durchgeführt werden. Dabei werden geeignete Mengen an Ammoniak oder Mono- oder Dialkylamin eingesetzt, die vorzugsweise bei ungefähr 0,5 bis ungefähr 50 Gewichtsteilen, besonders bevorzugt bei ungefähr 1 bis ungefähr 20 Gewichtsteilen, jeweils bezogen auf 100 Gewichtsteile der zur Hydrierung vorgesehenen Verbindung oder Verbindungen, eingesetzt. Besonders bevorzugt werden wasserfreier Ammoniak oder wasserfreie Amine eingesetzt.

Für Oxidationen wird im allgemeinen Luft oder reiner Sauerstoff verwendet. Für Dehydrierungen werden die üblicherweise verwendeten Kohlenwasserstoffe, insbesondere Methan bzw. Erdgas, eingesetzt.

Die Erfindung wird im folgenden anhand von einigen Ausführungsbeispielen näher erläutert, wobei sich Beispiele 1 bis 4 auf die Hydrierung einer aromatischen Verbindung, in der mindestens eine Hydroxylgruppe an einen aromatischen Kern gebunden ist, beziehen. Beispiele 5 bis 7 beziehen sich auf die Hydrierung einer aromatischen Verbindung, in der mindestens eine Aminogruppe an einen aromatischen Kern gebunden ist. Beispiele 8 bis 12 beziehen sich auf die Umsetzung von C=O-Gruppen enthaltende Verbindungen, Beispiele 13 bis 16 betreffen die Umsetzung von Polymeren.

### BEISPIELE

### Herstellung des Katalysators 1

Ein meso-/makroporöser Aluminiumoxidträger in Form von 4 mm-Extrudaten, der eine BET-Oberfläche von 238 m²/g und ein Porenvolumen von 0,45 ml/g besaß, wurde mit einer wäßrigen Ruthenium-(III)-nitrat-Lösung, die eine Konzentration von 0,8 Gew.-% aufwies, getränkt. 0,15 ml/g (ungefähr 33 % des Gesamtvolumens) der Poren des Trägers besaßen einen Durchmesser im Bereich von 50 nm bis 10.000 nm und 0,30 ml/g (ungefähr 67% des Gesamt Porenvolumens) der Poren des Trägers wiesen einen Porendurchmesser im Bereich von 2 bis 50 nm auf. Das während des Tränkens vom Träger aufgenommene Lösungsvolumen entsprach dabei in etwa dem Porenvolumen des verwendeten Trägers.

Anschließend wurde der mit der Ruthenium-(III)-nitrat-Lösung getränkte Träger bei 120°C getrocknet und bei 200°C im Wasserstrom aktiviert (reduziert). Der so hergestellte Katalysator enthielt 0,05 Gew.-% Ruthenium, bezogen auf das Gewicht des Katalysators.

### Beispiel 1

Es wurde eine 50 Gew.-%ige Lösung von p-tert.-Butylphenol in THF hergestellt. Anschließend wurden 2500 g/h dieser Lösung mit Wasserstoff bei 180°C und einem Gesamtdruck von 2,6 · 10⁷ Pa durch einen Durchflußreaktor, der mit 3,2 1 des Ru-Katalysators 1 gefüllt war, geleitet. Nach dem Abdestillieren des Lösungsmittels besaß das Hydrierungsprodukt folgende Zusammensetzung:
- 99,9% cis,trans-4-tert.-Butylcyclohexanol
- < 0,01% p-tert.-Butylphenol

### Beispiel 2

Die Hydrierung wurde in der gleichen Weise wie in Beispiel 1 beschrieben durchgeführt, wobei jedoch 3500 g der 50 Gew.-%igen p-tert.-Butylphenol-Lösung in THF bei 200°C durch den Reaktor geleitet wurden. Nach dem Abdestillieren des Lösungsmittels besaß das Hydrierungsprodukt folgende Zusammensetzung:
- 99,8% cis,trans-4-tert.-Butylcyclohexanol
- < 0,01% p-tert.-Butylphenol

### Beispiel 3

Die Hydrierung wurde in der gleichen Weise wie in Beispiel 1 angegeben durchgeführt, es wurde jedoch eine 50 Gew.-%ige Lösung von p-tert.-Butylphenol in i-Butanol eingesetzt. Nach dem Abdestillieren des Lösungsmittels besaß das Hydrierungsprodukt folgende Zusammensetzung:
- 67,5% trans-4-tert.-Butylcyclohexanol
- 32,4% cis-4-tert-.-Butylcyclohexanol
- < 0,01% p-tert.-Butylphenol

### Beispiel 4

In einem 3,5 l-Autoklaven wurden 2 kg einer Lösung von 50 Gew.-% Bisphenol A in THF und 500 ml des Katalysators aus Beispiel 1 in einen Katalysatorkorb gegeben. Anschließend wurde diskontinuierlich bei 150°C und 2 x 10⁷ Pa fünf Stunden lang hydriert. Der Umsatz zum gewünschten cycloaliphatischen Diol-Isomerengemisch war quantitativ, und der Restaromatengehalt betrug weniger als 0,01%.

### Beispiel 5

1,2 l des wie oben beschrieben hergestellten Katalysators 1 wurden in einen elektrisch beheizbaren Durchflußreaktor eingefüllt. Anschließend wurde ohne vorhergehende Aktivierung bei 2 x 10⁷ Pa und 160°C mit der Hydrierung von Anilin begonnen. Die Hydrierung wurde kontinuierlich in Sumpffahrweise ausgeführt, wobei ein Teil des Hydrierungsaustrags über eine Umlaufpumpe rückgeführt und dem Einsatzstoff vor dem Reaktor zugemischt wurde. Bezogen auf die Menge an Anilin wurde so die zehnfache Menge an Hydrierungsprodukt als Lösungsmittel zugesetzt. Am Kopf des Abscheiders wurden 500 bis 600 l H₂/h entspannt. Die kontinuierlich dem Reaktor zugeführte Anilinmenge entsprach einer Katalysatorbelastung von 0,6 kg/l x h.

In Abhängigkeit von den Reaktionstemperaturen ergaben sich bei stationären Reaktionsbedingungen folgende Produktzusammensetzungen:

| **Temperatur** **(°C)** | **CHA¹⁾** **(%)** | **DCHA²⁾** **(%)** | **Anilin** **(%)** | **Cyclohexan** **+ Cyclohexen (%)** |
|---|---|---|---|---|
| 160 | 99,1 | 0,45 | 0,10 | 0,04 |
| 180 | 97,0 | 2,75 | 0,06 | 0,06 |
| 200 | 95,9 | 3,9 | - | 0,09 |

| | | | | |
|---|---|---|---|---|
| ¹⁾ CHA = Cyclohexylamin; | | | | |
| ²⁾ DCHA = Dicyclohexylamin | | | | |

### Beispiel 6

Die Hydrierung wurde wie in Beispiel 5 beschrieben durchgeführt, wobei jedoch zusätzlich kontinuierlich wasserfreier Ammoniak eindosiert wurde.

Bezogen auf 100 Gew.-% an Anilin wurden 10 Gewichtsteile Ammoniak zugesetzt. In Abhängigkeit von den Reaktionstemperaturen ergaben sich bei stationären Reaktionsbedingungen folgende Produktzusammensetzungen:

| **Temperatur** **(°C)** | **CHA¹⁾** (%) | **DCHA²⁾** **(%)** | **Anilin** **(%)** | **Cyclohexan** **+ Cyclohexen (%)** |
|---|---|---|---|---|
| 180 | 99,3 | 0,08 | - | 0,07 |
| 200 | 98,4 | 0,8 | - | 0,09 |

| | | | | |
|---|---|---|---|---|
| ¹⁾ CHA = Cyclohexylamin; | | | | |
| ²⁾ DCHA = Dicyclohexylamin | | | | |

### Beispiel 7

In einem 3,5 l-Autoklaven wurden 2 kg einer Lösung von 50 Gew.-% Toluylendiamin(2,4-;2,6-Diaminotoluol-Isomerengemisch) in THF und 500 ml des oben beschriebenen Katalysators gegeben. Anschließend wurde diskontinuierlich bei 150°C und 2 x 10⁷ Pa fünf Stunden lang hydriert. Der Umsatz zum gewünschten cycloaliphatischen Diamin-Isomerengemisch war quantitativ, und der Restaromatengehalt war geringer als 0,01%.

### Beispiel 8 (Vergleich)

In einem Rohrreaktor (1 = 2500 mm, Ø = 45 mm) wurden 3 l Katalysator 1 eingebracht, der Reaktor mit n-Butanol gefüllt und bei 3 · 10⁶ Pa (30 bar) Wasserstoffdruck auf 180 °C aufgeheizt. Anschließend wurde bei einer Umlaufmenge von 50 l/h kontinuierlich eine Menge von 1 kg/h n-Butyraldehyd in den Reaktor gefahren. Der erhaltene Reaktionsaustrag war farblos und frei von Ruthenium.

Die gaschromatographische Auswertung ergab einen Umsatz von 99,4% und eine Selektivität bezüglich n-Butanol von 99,7%, jeweils bezogen auf die eingesetzte Menge von n-Butyraldehyd.

### Beispiel 9 (Vergleich)

In einen 3,5 l-Autoklaven wurden 3 l Katalysator 1 sowie 700 g eines Ethylen/CO-Copolymeren (Mw = 5.000, CO-Gehalt 35%), gelöst in 1.300 g THF, eingefüllt.

Anschließend wurde der Ansatz bei 180 °C und 2 · 10⁷ Pa (200 bar) Wasserstoffdruck 5 h lang hydriert. Der Umsatz zum gewünschten Polyalkohol lag bei 93%, bezogen auf die eingesetzte Menge des Copolymeren.

### Beispiel 10

In einem 3,5 l-Autoklaven wurden 3 l Katalysator 1 eingesetzt und 2.000 g Benzaldehyd eingefüllt. Anschließend wurde der Ansatz bei 180 °C und 2 · 10⁷ Pa (200 bar) Wasserstoffdruck 10 h lang hydriert. Der Umsatz zum gewünschten Cyclohexylmethanol lag bei 100% bei einer Selektivität von 96,2%, jeweils bezogen auf die eingesetzte Menge an Benzaldehyd.

### Beispiel 11 (Vergleich)

In einem 3,5 l-Autoklaven wurden 3 l Katalysator 1 eingesetzt und 2.000 g 2-Ethylhexanal eingefüllt. Anschließend wurde der Ansatz bei 180 °C und 2 · 10⁷ Pa (200 bar) Wasserstoffdruck 10 h lang hydriert. Der Umsatz zum gewünschten 2-Ethylhexanol lag bei 100% bei einer Selektivität von 97,2%, jeweils bezogen auf die eingesetzte Menge an 2-Ethylhexanal.

### Beispiel 12 (Vergleich)

In einem 0,3 l-Rührautoklaven wurden 100 ml Adipinsäuredimethylester in Gegenwart des Katalysators 1 umgesetzt. Der Ansatz wurde 12 h lang bei einem Wasserstoffdruck von 2 · 10⁷ Pa (200 bar) und einer Temperatur von 220 °C gerührt. Die gaschromatographische Analyse des Reaktionsaustrages ergab einen Umsatz von 98% und eine Hexandiol-Ausbeute von 91%, jeweils bezogen auf die eingesetzte Menge an Adipinsäuredimethylester.

## Patentansprüche

1. Verfahren zur Hydrierung von organischen Verbindungen ausgewählt aus aromatischen Verbindungen in Gegenwart eines Katalysators, der als Aktivmetall Ruthenium alleine in einer Menge von 0,01 bis 30 Gew.-%, bezogen auf das Gesamtgewicht des Katalysators, aufgebracht auf einem Träger, umfasst, **dadurch gekennzeichnet, dass** 10 bis 50 % des Porenvolumens des Trägers von Makroporen mit einem Porendurchmesser im Bereich von 50 nm bis 10.000 nm und 50 bis 90 % des Porenvolumens des Trägers von Mesoporen mit einem Porendurchmesser im Bereich von 2 bis 50 nm gebildet werden, wobei sich die Summe der Porenvolumina zu 100 % addiert, wobei das Verhältnis zwischen Metalloberfläche und der Trägeroberfläche, gemessen nach BET, im Bereich 0,0005 bis 0,3 liegt und der Träger Aluminiumoxid ist.

2. Verfahren nach Anspruch 1, **dadurch gekennzeichnet, dass** die organische Verbindung ausgewählt wird aus der Gruppe bestehend aus einer aromatischen Verbindung, in der mindestens eine Hydroxylgruppe an einen aromatischen Kern gebunden ist, einer aromatischen Verbindung in der mindestens eine Aminogruppe an einen aromatischen Kern gebunden ist und einem Gemisch aus zwei oder mehr davon.

3. Verfahren nach Anspruch 2, **dadurch gekennzeichnet, dass** die aromatische Verbindung ausgewählt ist aus der Gruppe bestehend aus (Mono)alkylphenolen, wobei der Alkylrest in o-, m- oder p-Position zur Hydroxylgruppe stehen kann, bevorzugt 4-tert.-Butylphenol; aromatischen Aminen und aromatischen Diaminen, bevorzugt Anilin, Naphthylamin, Diaminobenzole, Diaminotoluole und Bis-p-aminophenylmethan.

4. Verfahren nach einem der vorstehenden Ansprüche, **dadurch gekennzeichnet, dass** die Umsetzung in Gegenwart eines Lösungs- oder Verdünnungsmittels durchgeführt wird.

## Claims

1. A process for hydrogenating organic compounds selected from amongst aromatic compounds in the presence of a catalyst comprising as active metal ruthenium alone in an amount of from 0.01 to 30% by weight, based on the total weight of the catalyst, applied to a support, wherein from 10 to 50% of the pore volume of the support is formed by macropores having a pore diameter in the range from 50 nm to 10,000 nm and from 50 to 90% of the pore volume of the support is formed by mesopores having a pore diameter in the range from 2 to 50 nm, with the sum of the pore volumes adding up to 100%, wherein the ratio between metal surface area and the support surface area, measured by the BET method, is in the range from 0.0005 to 0.3. and the support is aluminium oxide.

2. A process as claimed in claim 1, wherein the organic compound is selected from the group consisting of aromatic compounds in which at least one hydroxyl group is bonded to an aromatic ring, aromatic compounds in which at least one amino group is bonded to an aromatic ring and mixtures of two or more thereof.

3. A process as claimed in claim 2, wherein the aromatic compound is selected from the group consisting of (mono)alkylphenols, where the alkyl radical can be in the o, m or p positon relative to the hydroxyl group, preferably 4-tert-butyl-phenol; aromatic amines and aromatic diamines, preferably aniline, naphthylamine, diamino-benzenes, diaminotolueries and bis-p-aminophenyl-methane.

4. A process as claimed in any of the preceding claims, wherein the reaction is carried out in the presence of a solvent or diluent.

## Revendications

1. Procédé d'hydrogénation de composés organiques choisis parmi des composés aromatiques en présence d'un catalyseur qui comprend à titre de métal actif du ruthénium seul en une quantité allant de 0,01 à 30 % en poids par rapport au poids total du catalyseur, déposé sur un support, **caractérisé en ce que** 10 à 50 % du volume des pores du support sont formés par des macropores ayant un diamètre de pores dans la plage allan de 50 nm à 10 000 nm et de 50 à 90 % du volume des pores du support son formés par des mésopores ayant un diamètre de pores dans la plage allant de 2 à 50 nm, la somme des volumes des pores s'additionnant pour donner 100 %, le rapport entre la surface métallique et la surface du support, mesuré d'après BET, se situe dans la plage allant de 0,0005 à 0,3 et le support est l'oxyde d'aluminium.

2. Procédé selon la revendication 1, **caractérisé en ce que** le composé organique est choisi parmi le groupe constitué d'un composé aromatique dans lequel au moins un groupe hydroxyle est lié à un cycle aromatique, d'un composé aromatique dans lequel au moins un groupe amino est lié à un cycle aromatique et d'un mélange de deux ou plus de ceux-ci.

3. Procédé selon la revendication 2, **caractérisé en ce que** le composé aromatique est choisi parmi le groupe constitué des (mono)alkylphénols, le résidu alkyle pouvant se situer en position o, m ou p par rapport au groupe hydroxyle, de préférence le 4-tert.-butylphénol ; des amines aromatiques et des diamines aromatiques, de préférence l'aniline, la naphtyl-amine, les diaminobenzènes, les diaminotoluènes et le bis-p-aminophénylméthane.

4. Procédé selon l'une quelconque des revendications précédentes, **caractérisé en ce que** la conversion est réalisée en présence d'un agent de dissolution ou de dilution.
